# EUROPÄISCHE PATENTSCHRIFT

(11) **EP 0 993 309 B1**
(45) Veröffentlichungstag und Bekanntmachung des Hinweises auf die Patenterteilung: **05.09.2007**
(21) Anmeldenummer: 99927714.8
(22) Anmeldetag: 22.04.1999
(51) Int. Cl.: A61L 2/26

(54) **MODULARES STERISET**
MODULAR STERILE SET (STERISET)
ENSEMBLE STERILISE (STERISET) MODULAIRE

(30) Priorität: 30.04.1998 DE 19819328
(43) Veröffentlichungstag der Anmeldung: 19.04.2000
(60) Teilanmeldung: 06023501.7
(73) Patentinhaber: Thurgauer Kantonalbank, 8570 Weinfelden (CH)
(72) Erfinder: Draenert, Klaus, 81545 München (DE)
(74) Vertreter: Rentsch & Partner
(86) Internationale Anmeldenummer: PCT/DE1999/001222
(87) Internationale Veröffentlichungsnummer: WO 1999/056795

(56) Entgegenhaltungen:
- WO-A-94/01055
- DE-A- 4 138 674
- DE-A- 19 611 680
- US-A- 4 643 303
- US-A- 5 061 018
- US-A- 5 540 901

## Beschreibung

Die vorliegende Erfindung betrifft ein Modulsystem aus reversibel anordnenbaren Haltern auf Lochblechen, die wiederum in modularer Bauweise so zusammengesetzt werden können, dass am Ort der Anwendung kürzestmögliche Wege und bestmögliche Übersicht über die Anordnung einzelner Instrumente erreicht werden kann. Dies ist dadurch notwendig geworden, da sich in der Vergangenheit vor allem bei chirurgischen Eingriffen gezeigt hat, dass einzelne wichtige Instrumente, die normalerweise vorverpackt in einem Sterilisationsset, welches für eine bestimmte Operation gerichtet worden war, fehlten, was aus dem Grunde nicht gleich erkannt worden war, da normalerweise die Instrumente zwar nach Checkliste, aber ungeordnet in sog. "Sieben" im Sterilisierbehälter sterilisiert werden und erst danach auf dem Tisch nach einer bestimmten Ordnung aufgebaut werden.

Normalerweise steht die Instrumentierschwester zwischen dem Instrumententisch auf der einen Seite und dem Operationstisch auf der anderen Seite.

Das Aufbauen der Instrumente erfolgt nach dem Herausnehmen aus den Sterilisationsboxen

Das übersichtliche Anordnen wird nach einer bestimmten erfahrungsgemäss erarbeiteten Reihenfolge vorgenommen: Die Instrumente werden auf den Operationstischen, die wie eine Wagenburg um die Operationsschwester aufgebaut sind, angeordnet und meist über den Operationsflur dem Chirurgen an den Operationstisch angereicht. Das Aufbauen der Tische und das übersichtliche Anordnen der Instrumente benötigt meist eine Vorlaufzeit von mindestens einer halben Stunde. Passiert es nun, dass beim Herüberreichen eines wichtigen Instrumentes, welches auch noch eine gewisse Schlüpfrigkeit hat, beispielsweise eines Knochenbohrers, dieser der Schwester entgleitet und auf die unsterile Unterlage fällt, so verliert der Chirurg heute bis zu einer Stunde Zeit. In der Vergangenheit wurden bei solchen Zwischenfällen die Instrumente gereinigt und blitzsterilisiert, was aufgrund der Unsicherheiten der kurzen Sterilisiervorgänge nunmehr nicht mehr erlaubt ist. Die sorgfältige Validierung einzelner Operationen hat gezeigt, dass sowohl vom Ablauf der Operation her als auch von der Ergonomie der Instrumentierwege die Arbeiten, die ausschliesslich von der Schwester verrichtet werden, besser konzentriert sind in unmittelbarer Nähe der Operationsschwester, während alle die Instrumente, die auch vom Chirurgen benutzt werden, besser in unmittelbarer Nähe des Chirurgen angeordnet sind, so dass er ggf. auch durch direkten Zugriff sich des Instrumentes bedienen und dieses auch geordnet wieder an seinen vorherigen Platz zurücklegen kann. Für eine solche subtile, nach ergonomischen Gesichtspunkten erarbeitete Anordnung von Instrumenten und Geräten, einschliesslich der Energieversorgung (in der Regel Druckluft) gab es bisher keine zufriedenstellende Vorrichtung, die es erlaubt hätte, in modularer Weise die notwendigen Instrumente, in optimierter Form anzuordnen. Hinzu kommt, dass die relativ aufwendige, optimierte Anordnung von Instrumenten und Geräten nicht in unmittelbaren Zusammenhang mit der Operation, bei der sowohl die Operationsschwester als auch der Chirurg von anderen Prioritäten besetzt sind, optimal durchgeführt werden kann, sondern vielmehr besser am Tag zuvor nach dem Reinigen der Instrumente vorgenommen werden sollte. Im modularen Sterilisationsset angeordnet, werden die Instrumente gleich in fertiger Anordnung sterilisiert und in sog. "modulen Stecksystemen" unmittelbar am Operationstisch auf einem speziellen Instrumentiertisch individuell angeordnet. Die gesamte Zeit des Aufbaus verkürzt sich dadurch auf etwa 5 *-* 10 Minuten. Das Problem konnte in sehr einfacher Weise durch solche fein abgestimmten Modulsysteme gelöst werden, die der folgenden Erfindung zugrunde liegen.

### Stand der Forschung

Der Stand der Forschung und der angewandten Praxis in den Instrumentierzentren, wie Operationssälen oder ambulanten Praxen, ist der, dass die Instrumente für eine Operation auf freien nicht strukturierten Operationstischen, welche mit Operationstüchern abgedeckt werden, aufgebaut werden, zum Teil einzeln oder in einer Serie von gleichen Instrumenten direkt auf den Tisch oder in einzelnen geöffneten und steril abgedeckten Sterilisationsboxen, die einzeln oder nebeneinander aufgebaut werden und die darin enthaltenen Instrumente einsehen lassen.

Aus der US 4,643,303 ist eine Vorrichtung zum Sterilisieren, Transportieren und Lagern einer Vielzahl von chirurgischen Instrumenten bekannt, die.einen Drahtkorb umfasst, der von einem schachtelförmigen, mit einem Deckel verschliessbaren Behältnis aufgenommen werden kann. Im Drahtkorb lassen sich eine Mehrzahl von Instrumenten-Träger-Modulen anordnen, die derart geformt sind, dass sie jeweils eine Mehrzahl von identischen Instrumenten halten können. Die Träger-Module weisen eine im wesentlichen rechteckige Grundfläche auf und .können variabel im ebenfalls rechteckigen Drahtkorb angeordnet werden. Die Träger-Module weisen derart ausgebildete Instrumenten-Aufnahmeaufnehmungen auf, die es erlauben die Instrumente zur Vorbereitung einer Operation aus einer platzsparenden Sterilisierstellung in eine entsprechende Entnahmestellung im Trägermodul anzuordnen.

Ein weiteres modulares System zum Sterilisieren von medizinischen Instrumenten ist in der US 5,540,901 beschrieben. Ein fester, im wesentlichen quaderförmiger Behälter ist mit einer Mehrzahl von vertikalen Lochreihen in den Seitenwänden versehen. Die Löcher weisen alle eine einheitliche Grösse auf und dienen der lösbaren Befestigung von Fixierelementen. Mittels der Fixierelemente lassen sich ein oder mehrere Ablagen für Instrumente in vordefinierten Positionen im Inneren des Behälters anbringen.

In der WO94/01055 ist ein modulares Kassettensystem zur Sterilisation von Zahnmedizinischen Instrumenten beschrieben. Eine Bodenplatte und ein Deckel sind an den einander zugewandten Seiten mit einem dichten Raster von hohlzylindrischen Nocken versehen. Im Nockenraster an Basis und Deckel lassen sich verschiedene Instrumentenhalter platzieren, die bei geschlossener Kassette derart zusammenwirken, dass verschiedene dentalchirurgische Instrumente fest zwischen den oberen und unteren Haltern klemmend gehalten sind.

Vereinzelt gibt es weiter strukturierte sog. "Sterilisationssiebe", die einzeln unterteilt sind, so dass einzelne Fächer entstehen, in denen bestimmte Instrumente oder Implantate übersichtlich angeordnet abgelegt werden können. Vereinzelt, z. B. im Instrumentenkatalog der Firma Martin Medizin-Technik in Tuttlingen, Ausgabe 8/97, gibt es für die aus Lochblechen gefertigten Sterilisationssiebe Metallstifte, sogenannte "Sortierstifte" mit Artikelnummer 55 991 09 MA, die an einem Ende eingefräst sind, so dass eine komprimierbare Metallspange entsteht, die man zusammendrücken kann; in diesem Zustand lassen sich die Stifte in die Lochbleche einstecken. Bei einem solchen Stift lassen sich einzelne Instrumente auffädeln; durch die Anwendung von drei Stiften, in entsprechender Anordnung lassen sich auch einzelne gerade oder leicht gekrümmte Instrumente oder Implantate lose in einer bestimmten Anordnung plazieren. Keinesfalls ist es aber möglich, mit diesem System in übersichtlicher Weise verschiedene Instrumente in fester Anordnung unterzubringen, schon gar nicht die Vielfalt von Instrumenten, die für eine Operation, benötigt werden. Darüber hinaus wird die Anwendung der Stifte dadurch erschwert, dass sie sich kaum oder nur schwer im Blech verankern lassen.

Keines der vorhandenen Systeme ist in der Weise stapelbar, dass Instrumente in fester und vorgegebener Anordnung miteinander verbunden werden können, so dass ein geschlossenes Herausnehmen aus dem Sterilisationsgefäss möglich ist. Auf diese Weise wird ein Instrumentierweg beim Richten des Instrumentierarbeitsplatzes gespart.

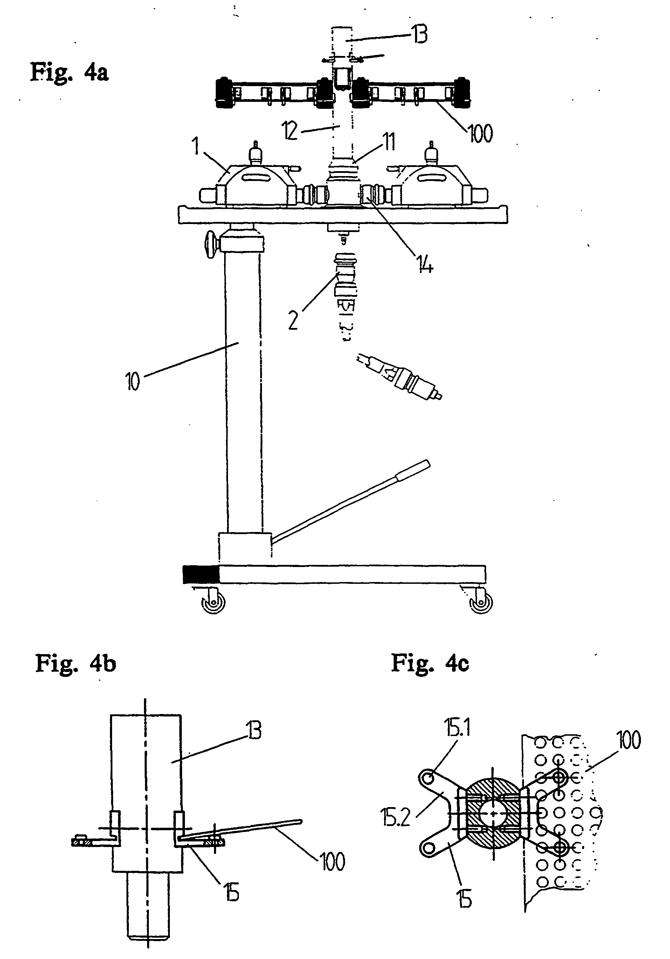

Am einfachsten zu verstehen ist die Erfindung, die alle diese Probleme sehr einfach und elegant löst beim Durchführen einer Hüftgelenkersatzoperation: beispielsweise benötigt man Geräte zur Erzeugung des Vakuums in unmittelbarer Nähe des Operationstisches; man benötigt Bohrmaschinen und Geräte zum Anmischen von Knochenzement und zum Applizieren von Knochenzement; daneben benötigt man Implantate und eine Reihe von Instrumenten, die zum Teil spezifischer, zum Teil allgemein chirurgischer Art sind. Das Aufbauen eines solchen Instrumentariums in Form der geschilderten Wagenburg um die Operationsschwester herum dauert mindestens 30 bis 40 Minuten. Wird nun eine solche Operation mit dem sog. "Modulsystem" vorbereitet, so sind bereits am Tag vor der Operation alle Instrumente in fester Position in reversibler Befestigung, aber für diese Operation fest angeordneten Weise, im Sterilisationsset untergebracht und in diesem sterilisiert. Sie können am Tag der Operation geordnet in dieser Weise herausgenommen und an einer modularen Säule befestigt werden, welche ebenfalls sterilisert werden kann, und in der Weise angeordnet ist, dass sie in einem Basismodul verankert wird, aus der gleichzeitig die Druckluftversorgung als Energieversorgung für die Betriebsmaschinen zum Bohren, Fräsen und Zementapplizieren genommen werden kann. Ein solcher Operationstisch ist in der Abbildung 4a dargestellt. Er besteht aus einem normalen U-förmigen Operationstisch mit einer zentralen Aufnahme für eine zentrale Säule (Fig. 4a). Das Tablett des Instrumententisches wird normal mit einem sterilen Sack steril abgedeckt. Dieser wird mit der zentralen Säule perforiert und das sterile und für Flüssigkeiten undurchdringbare Abdecktuch wird so zwischen Säule und Tisch eingeklemmt, dass keine Kolonisierung von sterilen Oberflächen mit Bakterien erfolgen kann. Der Operationstisch kann in derselben Weise auch durch einen doppelten Disposable-Abdecksack abgedeckt werden. Die Säule nimmt nicht nur die Antriebsmaschinen auf, sondern kann im Zentrum kleine und grössere Module aufnehmen, an denen wiederum fertig gepackte Instrumentensets, welche aus den Sterilboxen entnommen werden, angedockt sind und in übersichtlicher Weise angeordnet werden. In dieser Weise kann individuell für jede Hüftgelenkersatzoperation ein Set mit Implantaten von Pfannen und Femoralkomponenten und darüber ein oder zwei Sets mit spezifischen Instrumenten angeordnet werden. Das Besondere an diesem Modularsystem ist das, dass jedes einzelne Instrument dadurch, dass eine Art Baukastensystem von Haltern mit Aufnahmen für die Instrumente besteht, fest angeordnet werden kann. Wird ein Instrument für die Operation gerichtet, so ist es entsprechend der Reihenfolge der Anwendung in übersichtlicher Weise fest auf dem Lochbrett fixiert, gegebenenfalls beschriftet und zwischen Haltern fest gelagert, aus denen es herausgenommen werden kann. Ist dieses Instrument einmal benützt, so kann es beispielsweise auf einen der beiden Halter, die für die feste Anordnung des Instrumentes notwendig sind, in aufrechter Position aufgesteckt werden. Der Chirurg weiss sofort, was das zuletzt benutzte Instrument war und kann dementsprechend entweder dieses oder das im Operationschritt nächstfolgende Instrument einsetzen.

Wird während der Operation von der Schwester etwas vorbereitet, wie beispielsweise das Vakuummischen von Knochenzement, so kann der Chirurg dennoch ohne Zeitverlust weiter operieren, kann sein Operationsgebiet für den Eingriff, für den die Schwester den Knochenzement vorbereitet, sorgfältig präparieren. Die Schwester kann sich auf ihre Tätigkeit konzentrieren und der Chirurg ist für diesen Moment unabhängig von der Instromentierschwester und kann die einzelnen Instrumente sich selbst greifen und in derselben Weise auch selbst zurücklegen. Die Anordnung der Instrumente ist am Tag vor der Operation bereits festgelegt worden, in dem die Instrumente für den Chirurgen ihm nah und die, die von der Schwester gebraucht werden, auf der Seite der Schwester angeordnet sind. Alle Versorgungsleitungen sind kurz und zwar zwischen der zentralen Säule und dem Operationstisch bzw. zwischen der Einheit der Schwester und der zentralen Säule. Meist kommen diese Versorgungseinheiten ohne Schlauchsysteme aus und sind direkt angeflanscht (Fig. 4a). Die einzelnen Instrumentensets sind einfach auf die jeweiligen Module aufsteckbar. Die Länge der Module wird jeweils so gewählt; dass sie frei drehbar sind über den auf der Basisplatte des Instrumentiertisches angeordneten Geräte (Abb. 4b). Die Halter sind ohne irgendwelche Schraubverbindungen, so dass sie schnell eingehängt und eingesteckt werden können (Abb. 4c). Die am Vortag gerichteten Instrumente und am selben Nachmittag nach der Operation wieder hergerichteten Instrumente sind, nachdem sie gereinigt und von Rückständen befreit worden sind, übersichtlich und in der Reihenfolge, in der sie benützt werden, auf dem Lochbrett in fester Position angeordnet und können aus diesen Haltern sehr leicht herausgenommen und nach Gebrauch auch in anderer Position aufrecht aufgesteckt werden. Auf diese Weise ist es in sehr einfacher Anordnung möglich, ein sehr wichtiges instrument, welches gerne zu Boden fällt, in doppelter Ausgabe übersichtlich auf dem Instrumentierblech zu positionieren.

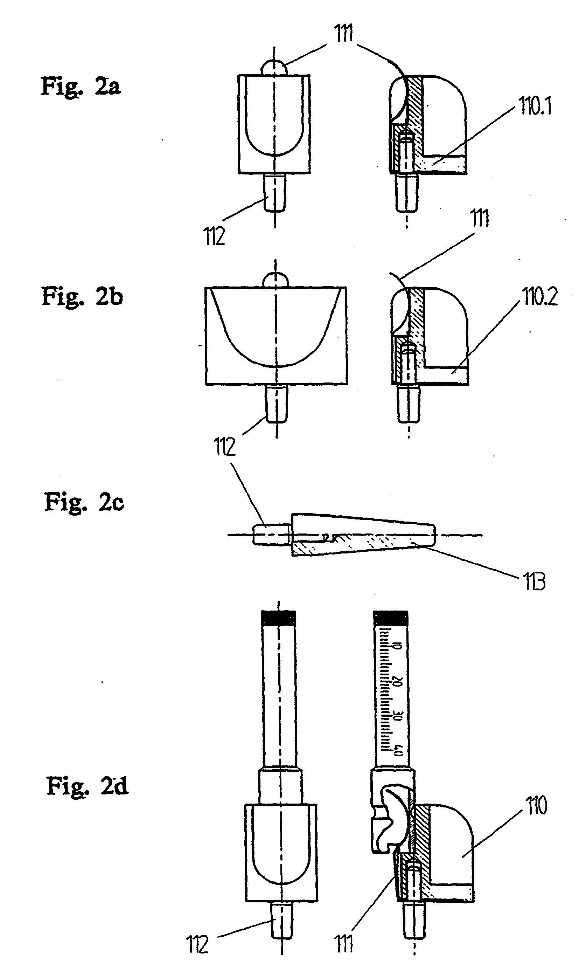

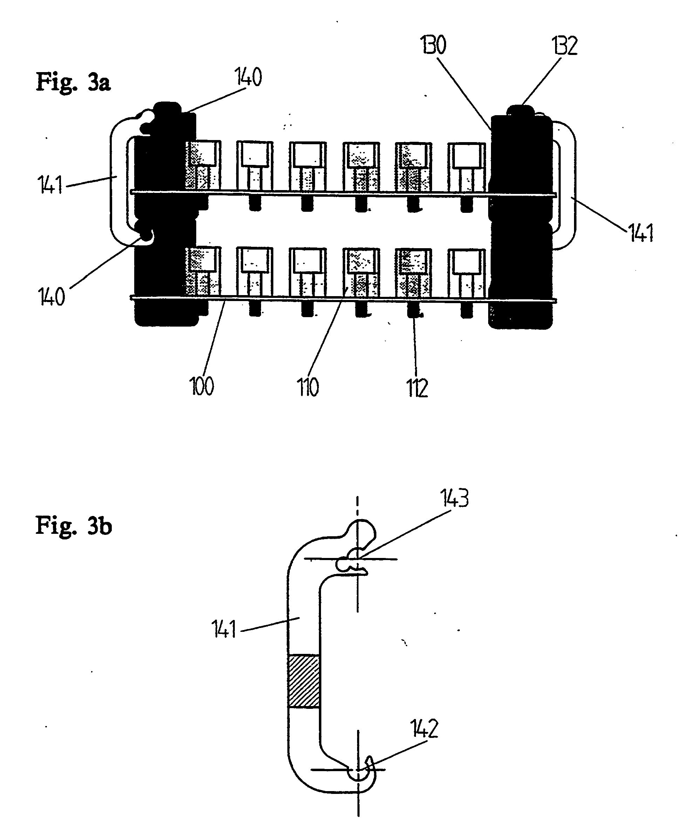

Das Risiko einer Operation wird umso kleiner, je sorgfältiger die Planung eines solchen Sets vorgenommen wurde und je vollständiger ein Packen des Sets validiert wurde, d.h. entsprechend einer Checkliste am Tag vor der Operation hingerichtet wurde. Die einzelnen modularen Halter für die Instrumente sind so beschaffen (Abb. 2a - d), dass jedes Instrument mit einem, meistens zwei Haltern fest fixiert werden kann. Instrumentensets, die zusammengehören und nacheinander benützt werden, oder auch nur um diese zusammen aus der Sterilbox zu entnehmen, da sie übereinander auf der zentralen Säule angeordnet werden, können auf sehr einfache Weise über die Griffleisten fest aneinander gekoppelt werden, so dass sie zusammen aus der Sterilbox entnommen werden können (Fig 3a und 3b). Ein besonderer Vorteil dieses modularen Systems ist es, dass es für jede Operation in freier Kombination so optimiert werden kann, dass aufgrund der verkürzten Instrumentierwege und aufgrund der vorgegebenen und vorgepackten Anordnung die Zeiten im Operationssaal zwischen 20 Minuten bis zu einer halben Stunde gekürzt werden können. Die Instrumentierung, dies hat sich gezeigt (Europilotstudie Zentrums für Orthopädische Wissenschaften, München, 1995 - 1998) war einer der wichtigten Faktoren Für die Verkürzung der Operationszeiten und für die Sicherheit des operativen Eingriffes, da jedes Improvisieren des Chirurgen aufgrund des Fehlens eines Instrumentes wegfällt.

### Beschreibung

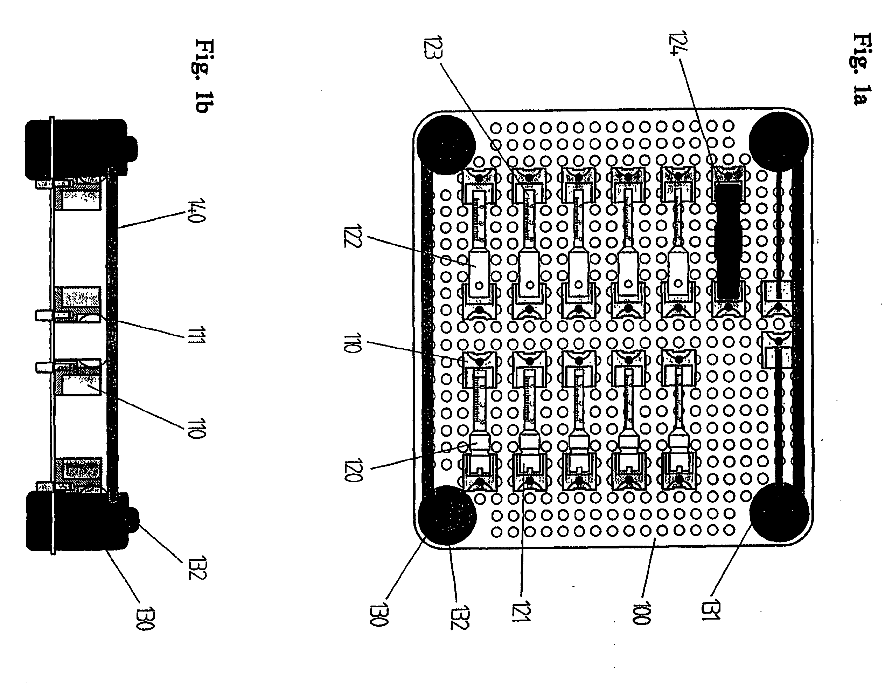

In der Abbildung 1a und 1b ist ein kleines Transplantationsset , bestehend aus Diamantfräsen (120) und den sogenannten Extraktoren (122) dargestellt. Mit einem Paar von diamantbeschichteten Fräsen (120) und (121) können freie Knorpel-Knochen-Transplantate durchgeführt werden. Ein solches Zwillingsinstrumentarium ist auf 1/10 mm aufeinander abgestimmt und das Verwechseln der äusserlich sehr ähnlichen Instrumente würde das Operationsergebnis zunichte machen. Die Tatsache, das die Instrumente in fester Reihenfolge angeordnet sind und die Tatsache, dass ein gebrauchtes Instrument am Ende des Halters in vertikaler Position aufgesteckt werden kann (Abb 2a), vermeidet diesen Fehler, da jedes Transplantat, wenn es mit dem Diamant geschliffen worden ist, mit einem dazu direkt korrelierenden Instrument, dem sogenannten Extraktor (122) herausgenommen wird und nur das Instrumentenpaar 120/122 aufeinander abgestimmt ist, resp. das Instrumentenpaar 121 / 123 u.s.w. Ein Verwechseln dieser Zuordnung würde zu dramatischen Komplikationen führen, indem Transplantat oder Transplantatbett zerstört würden. Der für die Extraktion notwendige Handgriff (124) ist übersichtlich in der passenden Grösse den Extraktoren zugeordnet für die dieser entsprechende Handgriff (124) passt. Durch entsprechende Anordnung von Eckmodulen (130), die in sich wiederum Aufnahmen für Instrumente beinhalten können (131), wird erreicht, dass die einzelnen Sterisets übereinander gestapelt werden können und in einfacher Weise an einer Griffleiste (140); auch in heissem Zustand; aus der Steribox herausgenommen werden können.

In der Abbildung 3a ist dargestellt, dass zwei solcher Griffleisten sehr leicht und sicher durch mindestens zwei Klammerhalter (141), welche in Schnappverschlüssen (140) einrasten, aneinandergekoppelt werden können. Die Klammerhalter (Abb 3b) sind so konstruiert, dass sie in der unteren Griffleiste eingehakt und in der oberen Griffleiste eingeschnappt werden, wobei ein kleiner Widerstand überwunden werden muss (142/143 der Abb 3b). Die sorgfältige Beobachtung von Instrumentierabläufen und Validierung der einzelnen Schritte liess erkennen, dass es sehr hilfreich ist, wenn das eben benutzte Instrument in anderer Position zurückgesteckt werden kann, was sehr einfach durch die Erfindung, welche in der Abbildung 2a (111) dargestellt ist, möglich ist. Auf diese Weise erkennt der Chirurg die Diamantfräse, mit der er das Transplantat entnommen hat, und kann so für die Vorbereitung des Transplantatbettes die nachfolgende Fräse (1a, 121) wählen. Erst wenn die heikle Phase der Operation, die Abstimmung von Transplantatbett und Transplantat erfolgreich beendet wurde, werden die Instrumente wieder in ihre Halter zurückgelegt. Besondere Instrumente, wie Scheren und andere allgemeine Instrumente werden vorteilhafterweise von Haltern aufgenommen, die in Abb 2c dargestellt sind. Auch Prothesen lassen sich vorteilhaft zwischen Haltern der Art wie in Abb 2a, 110.1 und Abb 2b, 110.2, respektive Abb 2c, 113, auch einzeln oder in Serien sicher positionieren. Ein fertig gerichtetes Instrumentenset wird notwendigerweise direkt in unmittelbarer Nähe des Operationsfeldes benötigt. In der Anordnung auf einem solchen Operationstisch Abb 4a-c (10) ist über die zentrale Säule (12 und 13) das Instrumentierset (100) frei drehbar über dem Gerät (1), sowohl für die Seite des Chirurgen, als auch für die Seite der Instrumentierschwester erreichbar.

Die Vorrichtungen gemäss der vorliegenden Erfindung sind gemäss einer vorteilhaften Ausführungsform der Erfindung so beschaffen, dass die Stützhalter zumindest paarweise verschiedene Farben haben und/oder Markierungen tragen. Die Vorrichtungen sind gemäss weiterer vorteilhafter Ausführungsformen zudem so beschaffen, dass die Stützhalter mindestens eine konkave, meist nach oben offene Aufnahme verschiedener Weite und Tiefe vorweisen, die eine sichere Aufnahme von Instrumenten ermöglicht.

Die Vorrichtungen sind gemäss weiterer vorteilhafter Ausführungsformen zudem so beschaffen, dass die Stützhalter Konen mit verschiedenen Durchmessern und Steigungen darstellen, die Instrumente mit Scherengriffen und Fingerhaltern aufnehmen können.

## Patentansprüche

1. Sterilisationssieb zum Sterilisieren und Bereitstellen von Instrumenten bestehend aus einer Basisplatte (100) mit Löchern für die Passage des Sterilisiermediums und mindestens zwei Einzelstützhaltern (110), welche aus einem Teil (112) zum reversiblen Befestigen in den Löchern der Basisplatte (100) und einem zentralen Körper (110. 1, 110.2) bestehen, so beschaffen in Grösse und Form, dass sie einzeln (113) und/oder in Kombination mit mindestens einem weiteren Stützhalter (110, 113, 130), zumindest ein Instrument fest aufnehmen können, indem die Basisplatte (100) hochragende Stützhalter (130) aufweist, **dadurch gekennzeichnet, dass** die Stützhalter (130) an mindestens zwei Enden mit zwei Rundstangen als Griffleisten (140) verbunden sind, über die mindestens zwei übereinander gestapelte Lochplatten (100) durch mindestens zwei freie Klammerhalter (141) miteinander verbunden werden können, die in die Griffleisten einhaken können zum Herausheben des oft noch heissen Sterilisationssiebes, und, dass die Löcher in der Basisplatte (100) eine Anordnung aufweisen, die ein Einklinken und eine reversible Fixation an einer Säule ermöglichen.

2. Sterilisationssieb nach Anspruch 1, so beschaffen, dass die Instrumente chirurgische Instrumente sind; die einzeln oder zumindest in zwei frei kombinierbaren Haltern angeordnet und von denen einzelne Halter so beschaffen sind, dass sie von ihrer operativen Anordnung die Instrumente tragen und auch Gebrauch dieselben in anderer Position aufnehmen (111).

3. Sterilisationssieb nach den Ansprüchen 1 oder 2, so beschaffen, dass die Einzelstützhalter (110.1), und/oder Stützhalter (130) zumindest paarweise verschiedene Farben haben und/oder Markierungen tragen.

4. Sterilisationssieb einem der Ansprüche 1 bis 3, so, beschaffen, dass die Stützhalter (110.1, 110.2) mindestens eine konkave, meist nach oben offene Aufnahme verschiedener Weite und Tiefe aufweisen, die eine sichere Aufnahme von Instrumenten ermöglicht.

5. Sterilisationsieb nach einem der Ansprüche 1 bis 4, so beschaffen, dass die Stützhalter Konen mit verschiedenen Durchmessern und Steigungen darstellen, die Instrumente mit Scherengriffen und Fingerhaltern aufnehmen können.

6. Sterilisationssieb nach einem der Ansprüche 1 bis 5, so beschaffen, dass der Befestigungsteil der Einzelstützhalter (112) zur reversiblen Fixation in der Lochplatte ein konischer Stift ist, der sich press- fit in der Lochplatte festsetzt.

7. Sterilisationssieb nach einem der Ansprüche 1 bis 6, so beschaffen, dass das Lochblech eine Aufnahme vorweist, über die es mit einem Halter an einer Säule reversibel verankert werden kann.

8. Sterilisationssieb nach einem der Ansprüche 1 bis 7, so beschaffen, dass es reversibel an einer Säule befestigt werden kann, die als Modul ausgebildet ist bestehend aus einem Körper als Säule (12) zur Aufnahme von mindestens einem Halter (15) für ein Lochblech (100) und einer Aufnahme für ein weiteres Modul (13) in seinem Kopfteil und einem Fussteil, mit welchem das Modul in einer zentralen Basissäule (11) verankert werden kann, welche so beschaffen ist, dass mindestens eine Versorgungsleitung für Druckluft mit Abluftkanälen enthalten ist und aus einem Fussteil und einem Körper besteht, dessen Fussteil nach der Sterilisation durch eine sterile Abdeckung hindurch reversibel in einer Aufnahme in einem Instrumententisch (10) verankert werden kann und einen Schnellkupplungsteil für die Druckluftzufuhr ausweist und dessen Körper mindestens eine Schnellkupplung (14) für das Anschliessen eines Druckluftschlauches beinhaltet.

## Claims

1. A sterilisation sieve for sterilising and preparing instruments, consisting of a base plate (100) with holes for the passage of the sterilisation medium, and at least two individual support holders (110), which consist of a part (112) for the reversible fastening in the holes of the base plate (100), and of a central body (110.1, 110.2), designed with regard to size and shape such that individually (113) and/or in combination with at least one further support holder (110, 113, 130), they may firmly receive at least one instrument, in that the base plate (100) comprises upwardly projecting support holders (130), **characterised in that** the support holders (130) are connected at at least two ends, to two round rods as grip ledges (140), via which at least two perforated plates (10) stacked over one another may be connected to one another by way of at least two free clip holders (141), which may hook into the grip ledges for lifting out the sterilisation sieve which is often still quite hot, and that the holes in the base plate (100) have an arrangement which permits the engagement and a reversible fixation on a column.

2. A sterilisation sieve according to claim 1, designed such that the instruments are surgical instruments, which are arranged individually or at least in two freely combinable holders, and of which the individual holders are designed such that they carry the instruments in their operative arrangement, and after use receive these in a different position.

3. A sterilisation sieve according to the claims 1 or 2, designed such that the individual support holders (110.1) and/or support holders (130) at least in pairs have different colours and/or carry markings.

4. A sterilisation sieve according to one of the claims 1 to 3, designed such that the support holders (110.1, 110.2) comprise at least one concave receiver, mostly open to the top, of a different width and depth, which permits the secure receiving of instruments.

5. A sterilisation sieve according to one of the claims 1 to 4, designed such that the support holders represent cones with different diameters and gradients, which may receive instruments which scissor grips and finger holders.

6. A sterilisation sieve according to one of the claims 1 to 5, designed such that the fastening part of the individual support holders (112), for the reversible fixation in the perforated plate, is a conical pin which locks in the perforated plate with a press fit.

7. A sterilisation sieve according to one of the claims 1 to 6, designed such that the perforated plate comprises a receiver, via which it may be reversibly anchored with a holder on a column.

8. A sterilisation sieve according to one of the claims 1 to 7, designed such that it may be reversibly fastened on a column which is designed as a module, consisting of a body as a column (12) for receiving at least one holder (15) for a perforated plate (100), and of a receiver for a further module (13) in its head part, and of a foot part with which the module may be anchored in a central base column (11) which is designed such that at least one supply conduit for pressurised air with discharge air channels is contained, and consists of a foot part and a body, whose foot part after sterilisation may be reversibly anchored through a sterile covering, in a receiver in an instrument table (10), and comprises a quick coupling part for the supply of pressurised air and whose body contains at least one quick coupling for the connection of pressurised air flexible tubing.

## Revendications

1. Tamis de stérilisation pour stériliser et mettre à disposition des instruments, constitué d'une plaque de base (100) dotée de trous pour le passage du milieu de stérilisation et d'au moins deux supports de soutien individuels (110) qui sont constitués d'une partie (112) pour la fixation réversible dans les trous de la plaque de base (100) et d'un corps central (110.1, 110.2), présentant une taille et une forme telles qu'ils peuvent recevoir fixement, individuellement (113) et/ou en combinaison avec au moins un autre support de soutien (110, 113, 130), au moins un instrument, en ce que la plaque de base (100) présente un support de soutien (130) dépassant verticalement, **caractérisé en ce que** le support de soutien (130) est relié à au moins deux extrémités avec deux tiges arrondies servant de barres de préhension (140) par le biais duquel au moins deux plaques à trous superposées (100) peuvent être reliées par au moins deux supports à brides libres (141), qui peuvent s'accrocher dans les barres de préhension pour soulever le tamis de stérilisation souvent encore chaud, et **en ce que** les trous de la plaque de base (100) présentent une disposition permettant un enclenchement et une fixation réversible à une colonne.

2. Tamis de stérilisation selon la revendication 1, conçu de telle sorte que les instruments sont des instruments chirurgicaux qui sont disposés individuellement ou au moins dans deux supports librement combinables, des supports individuels étant configurés de telle sorte qu'ils portent les instruments de par leur disposition fonctionnelle, et après utilisation, reçoivent ceux-ci dans une autre position.

3. Tamis de stérilisation selon les revendications 1 ou 2, conçu de telle sorte que le support de soutien individuel (110.1) et/ou le support de soutien (130) portent au moins différentes couleurs et/ou marquages par paires.

4. Tamis de stérilisation selon l'une quelconque des revendications 1 à 3, conçu de telle sorte que les supports de soutien 110.1, 110.2) présentent au moins un logement concave, ouvert la plupart du temps vers le haut, de différentes largeurs et profondeurs, permettant une réception fiable des instruments.

5. Tamis de stérilisation selon l'une quelconque des revendications 1 à 4, conçu de telle sorte que les supports de soutien représentent des cônes présentant différents diamètres et pas permettant de recevoir les instruments avec des anneaux de ciseaux et des supports pour les doigts.

6. Tamis de stérilisation selon l'une quelconque des revendications 1 à 5, conçu de telle sorte que la partie de fixation des supports de soutien individuels (112) est une pointe conique pour la fixation réversible dans la plaque à trous qui se fixe dans la plaque à trous par pression.

7. Tamis de stérilisation selon l'une quelconque des revendications 1 à 6, conçu de telle sorte que la plaque à trous présente un logement par le biais duquel elle peut être ancrée de façon réversible avec un support, à une colonne.

8. Tamis de stérilisation selon l'une quelconque des revendications 1 à 7, conçu de telle sorte qu'il peut être fixé de façon réversible à une colonne qui est constituée comme un module comprenant un corps en tant que colonne (12) pour recevoir au moins un support (15) pour une plaque à trous (100) et un logement pour un autre module (13) dans sa partie de tête et une partie inférieure avec laquelle le module peut être ancré dans une colonne de base centrale (11), qui est conçu de telle sorte qu'au moins une conduite d'alimentation d'air comprimé avec canaux d'évacuation d'air est contenue et est constitué d'une partie inférieure et d'un corps, la partie inférieure ou base pouvant être ancrée après la stérilisation par un couvercle de façon réversible dans un logement dans une table d'instruments (10) et présente une partie de couplage rapide pour l'admission d'air comprimé, le corps présentant au moins un couplage rapide (14) pour relier un tuyau d'air comprimé.
